# EUROPÄISCHE PATENTSCHRIFT

(11) **EP 0 808 184 B1**
(45) Veröffentlichungstag und Bekanntmachung des Hinweises auf die Patenterteilung: **21.10.1998**
(21) Anmeldenummer: 96903992.4
(22) Anmeldetag: 06.02.1996
(51) Int. Cl.: A61M 1/10

(54) **VORRICHTUNG ZUR UNTERSTÜTZUNG DER HERZTÄTIGKEIT MIT HILFE EINES BALLONS**
DEVICE FOR ASSISTING CARDIAC ACTIVITY BY MEANS OF A BALLOON
DISPOSITIF PERMETTANT DE SOUTENIR L'ACTIVITE CARDIAQUE A L'AIDE D'UN BALLONNET

(30) Priorität: 08.02.1995 DE 19504085
(43) Veröffentlichungstag der Anmeldung: 26.11.1997
(73) Patentinhaber: Sulzer Osypka GmbH, 79639 Grenzach-Wyhlen (DE)
(72) Erfinder: Osypka, Peter, Dr. Ing., 79639 Grenzach-Wyhlen (DE); Geistert, Wolfgang, 79619 Rheinfelden (DE)
(74) Vertreter: Schmitt, Hans, Dipl.-Ing.
(86) Internationale Anmeldenummer: EP9600480
(87) Internationale Veröffentlichungsnummer: WO9624395

(56) Entgegenhaltungen:
- EP-A- 0 547 733
- EP-A- 0 599 567
- WO-A-93/05827
- US-A- 4 014 317
- US-A- 4 016 871

## Beschreibung

Die Erfindung betrifft eine Vorrichtung zur Unterstützung der Herztätigkeit mit Hilfe eines in eine Hauptschlagader, insbesondere in die Aorta, eingelegten aufblasbaren und abblasbaren Ballons und einer Steuerung für eine Pumpe zum Aufblasen und Abblasen dieses Ballons in Übereinstimmung mit der Herztätigkeit derart, daß die durch das Aufblasen im Blutkreislauf erzeugte Druckwelle das Herz kurz nach dem Schluß der Aortenklappe erreicht und daß die durch das Abblasen des Ballons bewirkte negative Druckwelle die Aortenklappe zu dem Zeitpunkt erreicht, zu dem sie sich öffnet, wobei die Vorrichtung eine Einrichtung zur Erfassung der Herztätigkeit sowie eine Steuerung zum Verstärken und zur Synchronisation der Vorrichtung mit dem erfaßten aktuellen Herzrhythmus aufweist, wobei die Einrichtung zur Erfassung der Herztätigkeit in einem externen Herzschrittmacher angeordnet ist, der über Elektroden oder Herzdrähte an dem Herzen angeschlossen oder fixiert ist.

Eine derartige Vorrichtung ist aus der US-A-4 014 317 bereits bekannt.

Ferner kennt man aus der US-A.4 785 795 eine vergleichbare Vorrichtung, jedoch ohne externen Herzschrittmacher.

Eine derartige Vorrichtung wird als intraaortale Ballongegenpulsation (IABP) bezeichnet. Nach herzchirurgischen Operationen bestehen häufig Probleme bezüglich der Pumpleistung des Herzens. Eine Unterstützung der postoperativ unter Umständen zu schwachen oder noch zu schwachen Herzleistung besteht in der Anwendung der vorgenannten intraaortalen Ballongegenpulsation. Deren Prinzip besteht darin, daß der auf- und abblasbare längliche Ballon mit einer Kapazität von zum Beispiel etwa 80 Milliliter in die Hauptschlagader eingelegt und dort - wie vorstehend erwähnt - so betrieben wird, daß dem Herzen Kreislaufarbeit abgenommen wird.

Zu diesem Zweck wird der Ballon durch eine herzphasengesteuerte Pumpe zu einem solchen Zeitpunkt aufgeblasen, daß die dadurch erzeugte Druckwelle das Herz kurz nach dem Schluß der Aortenklappe erreicht. Diese Druckwelle erreicht das Innere der linken Herzkammer nicht, erhöht aber den Blutdruck in der Hauptschlagader, was eigentlich die Aufgabe des Herzens wäre, so daß das Blut in die Peripherie abströmt und den Körper des Patienten versorgt, also die Herztätigkeit unterstützt.

Weiterhin wird der Ballon wieder herzphasengesteuert zu einem solchen Zeitpunkt abgeblasen, daß die nunmehr hervorgerufene negative Druckwelle (Volumendefekt) die Aortenklappe erreicht, wenn diese sich öffnet, das Herz also beginnt, aus der linken Herzkammer Blut auszuwerfen. Das Herz hat dann nur gegen einen niedrigeren Druck anzupumpen und kann dadurch ein höheres Volumen an Blut auswerfen, wird also auch in dieser Phase durch die IABP unterstützt.

Anschließend wird der Ballon wieder aufgeblasen, das vom Herzen mit niedriger Energie aufgeworfene Blut unter Druck gesetzt und durchdringt den Körper besser, als dies ohne die IABP der Fall wäre. Diese Folge von Auf- und Abblasen im Zusammenwirken mit der Herztätigkeit vollzieht sich in einem ständigen Wechsel. Zusammenfassend bewirkt die IABP, daß das zu unterstützende Herz mit wenig Energie Blut auswerfen kann, das dann durch diese IABP gewissermaßen mit Energie "angereichert" wird.

Die dynamische Funktion der IABP, die als Pumpe keine eigenen Ventile aufweist, ist auf eine exakte Herzphasen-Synchronisierung für möglichst alle Herzschläge angewiesen. Hierzu ist in die Vorrichtung eine EKG-Steuerung eingebaut, die das Elektrokardiogramm (EKG) des Patienten von dessen Oberfläche ableitet, verstärkt, logisch bearbeitet und zur Synchronisation der Geräte mit dem aktuellen Herzrhythmus benutzt. Eine automatische Anpassung der Verstärkung an die Gegebenheiten des individuellen Elektrokardiogramms macht Empfindlichkeitseinstellungen an der IABP unnötig.

Nach herzchirurgischen Operationen bestehen häufig Rhythmusstörungen, die den Einsatz eines Herzschrittmachers, in der Regel eines externen Herzschrittmachers erforderlich machen. Zur Ermöglichung dieser Therapie werden während der Operation Elektroden, sogenannte Herzdrähte, an der Oberfläche des Herzens fixiert, die aus der Haut herausgeleitet und bei Bedarf mit einem externen Herzschrittmacher verbunden werden.

Mit modernen Herzschrittmachern kann eine differenzierte Rhythmusbehandlung erfolgen. Dabei kommen in Abhängigkeit von der zu behandelnden Rhythmusstörung ganz unterschiedliche EKG-Bilder oder -Kurven zustande. Die Stimulationsimpulse haben eine Amplitude von bis zu 20 Volt, wodurch das an der Oberfläche abgeleitete EKG, dessen maximale Amplitude normalerweise zwei Millivolt nicht übersteigt, so verzerrt wird, daß eine korrekte Synchronisation durch die Steuerlogik der IABP nicht mehr möglich ist (Übersteuerung). Es können die Bilder Vorhofstimulation, Kammerstimulation, Doppelstimulation, spontane Herzfunktion ohne Stimulation und atriale sowie ventrikuläre Extrasystolen in ständigem Wechsel vorliegen, so daß sich die EKG-Komplexe in Amplitude und Frequenzinhalt dauernd abrupt ändern. Die automatische Verstärkungsregelung (AGC) der IABP-Steuereinheit kann sich hierauf nicht einstellen. Eine korrekte IABP-Synchronisation wird in solchen Fällen illusorisch.

Da im postoperativen Verlauf häufig IABP und externe Herzschrittmacher am gleichen Patienten benutzt werden müssen, gibt es ebenso häufig die vorerwähnten und beschriebenen Schwierigkeiten im Zusammenwirken der beiden Vorrichtungen und Geräte, so daß ständig durch beobachtendes Personal eingegriffen werden muß. Bei sich ändernden Gegebenheiten muß durch manuelle Nachregelung an IABP und externem Herzschrittmacher ständig versucht werden, den best erreichbaren Zustand einzustellen.

Aus US-5 205 810 ist ein Verfahren bekannt, mit welchem Skelettmuskeln operativ so mit dem Herzen verbunden werden, daß sie die Herztätigkeit unterstützen können. Mit Hilfe eines Impulsgenerators werden die Skelettmuskeln stimuliert, um den Herzschlag zu unterstützen.

Aus der US 4 014 317 ist eine Vorrichtung bekannt, bei welcher die Pumpe und ein Herzschrittmacher in einem gemeinsamen Gehäuse zusammengefasst sind. Dies ergibt nicht nur ein aufwendiges und relativ sperriges Gerät, sondern ist auch unwirtschaftlich, weil der Herzschrittmacher nur zusammen mit der Pumpe Verwendung finden kann, also diese Vorrichtung nicht brauchbar ist, wenn nur ein Herzschrittmacher, aber keine Unterstützung der Herztätigkeit mit Hilfe eines Ballons benötigt wird. Zur Steuerung der Ballonpumpe dieses kombinierten Gerätes ist ein EKG-Gerät erforderlich, das wiederum von dem Herzschrittmacher getriggert werden muß.

Der Erfindung liegt deshalb die Aufgabe zugrunde, eine Vorrichtung der eingangs genannten Art zu schaffen, bei der bei Anwendung einer IABP und eines Herzschrittmachers dennoch eine bestmögliche Synchronisation zwischen IABP und Herztätigkeit erreicht werden kann, ohne daß manuell nachgeregelt werden muß. Die Triggerung der IABP mit einem Oberflächen-EKG soll vermieden werden.

Die Lösung dieser scheinbar widersprüchlichen Aufgabe besteht darin, daß die Einrichtung ein Zusatzmodul aufweist, das einen zusätzlichen Impuls für die Steuerung des Ballons erzeugt, der etwa Form und/oder Amplitude einer natürlichen, an der Körperoberfläche auftretenden R-Welle hat und über den EKG-Eingang einer Triggereinheit der Steuerung der Pumpe zuführbar ist, wobei dieser zusätzlich gebildete Impuls, der von dem Herzschrittmacher erfaßten oder der, von dem Herzschrittmacher, durch die Stimulation bestimmten Herzphase zeitlich zugeordnet ist.

Auf diese Weise kann der Herzschrittmacher, in der Regel ein externer Herzschrittmacher, erfassen und somit "wissen", was am Herzen geschieht. Der Herzschrittmacher "weiß", ob eine ventrikuläre Extrasystole stattgefunden hat, ob im Vorhof, in der Kammer oder in beiden stimuliert wurde oder ob das Herz im gesunden Rhythmus spontan arbeitet. Der Schrittmacher kann daher mit Vorteil den Impuls bilden, der der IABP die Information über die zeitlichen Verhältnisse des Herzens liefert. Das dem Schrittmacher abhängig von seiner konstruktiven Struktur (diskret, integriert, Hardware-, Softwareschrittmacher) zugeordnete Zusatzmodul kann einen Impuls erzeugen, der der vom Schrittmacher beobachteten oder der vom Schrittmacher durch Stimulation bestimmten Herzphase zeitlich fest zugeordnet ist.

Besonders vorteilhaft ist, daß der form- und amplitudenkonstante zusätzliche Impuls dem EKG-Eingang der IABP zugeführt und statt des Oberflächen-EKG des Patienten zur Triggerung der IABP benutzt werden kann. Unabhängig von den potentiell stark wechselnden Bildern im Oberflächen-EKG erhält also die Erkennungslogik der IABP bei der erfindungsgemäßen Ausbildung jetzt immer noch den im wesentlichen gleichen, form und/oder amplitudenkonstanten, zuverlässig herzphasengekoppelten Impuls, auf den sich die automatische Verstärkungsregelung (AGC) der IABP-Steuerung zuverlässig einstellen kann, so daß ein Eingreifen des Personals für eine Nachregelung an der IABP nicht mehr erforderlich ist.

Der von dem Herzschrittmacher beaufschlagte Zusatzmodul kann in die Steuerung der Vorrichtung integriert und über eine Leitung mit dem Herzschrittmacher verbunden sein. Dadurch ist das Zusammenwirken des Herzschrittmachers mit diesem Zusatzmodul und der Steuerung des Ballons bzw. der IABP auf einfache Weise sichergestellt, was im Zusammenhang damit zweckmäßig ist, daß ein Impuls zur Betätigung des Ballons durch vom Herzschrittmacher erfaßte oder stimulierte Herztätigkeit auslösbar ist.

Besonders zweckmäßig ist es dabei, wenn der Impuls bei Wahrnehmung einer spontanen Herzkammertätigkeit sofort und bei Stimulation der Kammer zeitversetzt, vorzugsweise um etwa 50 Millisekunden versetzt, erzeugbar ist. Somit kann der Zeitunterschied, der bei einer Stimulation der Kammer gegenüber einer spontanen Herzkammertätigkeit auftritt, berücksichtigt werden. Unabhängig davon, ob die Kammererregung spontan auftritt oder stimuliert wird, ist also der gebildete Impuls beiden Ereignissen phasengleich angekoppelt, wobei in besonders zweckmäßiger Weise ein externer Herzschrittmacher verwendbar ist. Ferner ist die Handhabung beim Anlegen der Vorrichtung an den Patienten besonders vorteilhaft.

Vor allem bei Kombination einzelner oder mehrerer der vorbeschriebenen Merkmale und Maßnahmen ergibt sich also gewissermaßen ein Herzschrittmacher, der Mittel zur Stimulation des Herzens und Mittel zur Wahrnehmung spontaner Herztätigkeit hat, welcher eine zusätzliche Einrichtung zur Bildung eines Impulses mit fester zeitlicher Koppelung an die Wahrnehmung spontaner ventrikulärer Herztätigkeit und fester zeitlicher, jedoch um einen bestimmten zeitlichen Betrag verschobener Koppelung an die Abgabe eines (ventrikulären) Stimulationsimpulses hat, wobei dieser zusätzlich gebildete Impuls zur Steuerung der Logik der IABP herangezogen wird, so daß die Synchronisation zwischen Herztätigkeit und IABP gewährleistet ist, wenn ein solcher externer Herzschrittmacher bei dem Patienten stimuliert.

Nachstehend ist ein Ausführungsbeispiel der Erfindung anhand der Zeichnung näher beschrieben. Es zeigt in schematisierter Darstellung:
- Fig.1: eine erfindungsgemäße Vorrichtung zur Unterstützung der Herztätigkeit mit Hilfe eines in die Hauptschlagader eingelegten aufblasbaren und abblasbaren Ballons mit einer Einrichtung zur Erfassung der Herztätigkeit, die in einem an das Herz angeschlossenen Herzschrittmacher angeordnet ist und ein Zusatzmodul zur Erzeugung eines Impulses für die Steuerung des Ballons aufweist,
- Fig.2: ein Beispiel eines Kurvenverlaufes des EKG eines rhythmusgesunden Patienten, bei welchem der Impuls bei Wahrnehmung der jeweiligen spontanen Herzkammertätigkeit sofort erzeugt werden kann,
- Fig.3: ein pathologisches EKG eines Herzkranken, bei welchem die Ballonpumpe Synchronisierungsprobleme hätte, da auf Grund der unterschiedlichen Amplituden der einzelnen Komplexe eine zuverlässige Triggerung nicht möglich ist,
- Fig.4: ein Beispiel eines EKG, bei welchem Rhythmusstörungen durch Elektrostimulation des Herzens behoben werden, wobei die entstehenden Elektroartefakte die Synchronisation der Ballonpumpe schwierig machen,
- Fig.5: eine EKG-Kurve eines an sich rhythmusgestörten Herzens, welches durch einen externen Herzschrittmacher "störungsfrei" gemacht ist, wobei der Schrittmacher auf einem Sonderkanal ein EKG-artiges Signal emittiert, das R-wellenartig immer gleich aussieht, so daß am Pumpeneingang keine Schwierigkeiten mit der Detektion auftreten sowie
- Fig.6: ein ähnliches Beispiel eines EKG wie in Fig.5, bei welchem "Pseudo-R-Wellen" für die Pumpe entweder bei Wahrnehmung einer ausreichenden spontanen Herzkammertätigkeit sofort oder während der Stimulation der Kammer zeitversetzt als Steuerimpuls zugeführt werden.

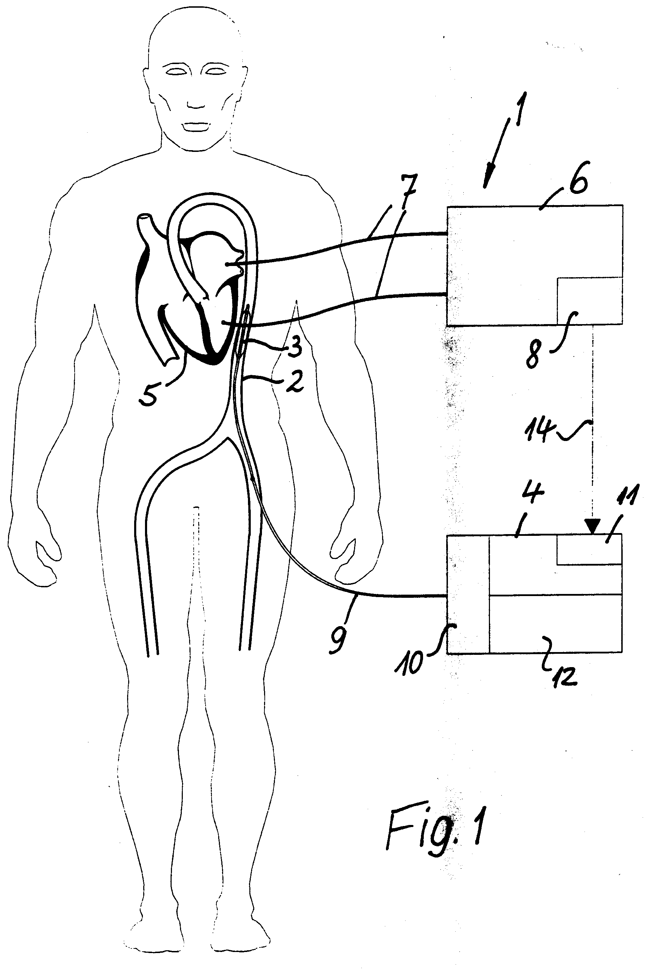

Eine in Fig.1 im ganzen mit 1 bezeichnete Vorrichtung, die dabei teilweise in der Art eines Blockschaltbildes dargestellt ist, dient zur Unterstützung der Herztätigkeit mit Hilfe eines in die Hauptschlagader 2 eingelegten, aufblasbaren und abblasbaren Ballons 3 und einer Steuereinheit 4 für das Aufblasen und Abblasen dieses Ballons 3 in Übereinstimmung mit der Herztätigkeit derart, daß die durch das Aufblasen im Blutkreislauf erzeugte Druckwelle das Herz 5 kurz nach dem Schluß der Aortenklappe erreicht und daß die durch das Abblasen des Ballons 3 bewirkte negative Druckwelle die Aortenklappe zu dem Zeitpunkt erreicht, zu dem sie sich öffnet, so daß also der Blutkreislauf jeweils auch bei schwacher Herztätigkeit sinnreich unterstützt wird.

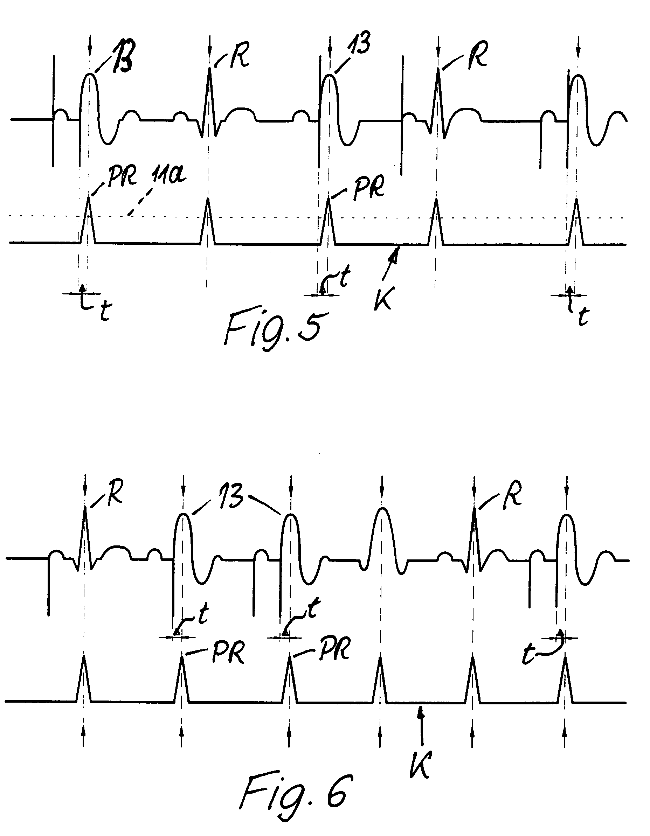

Die Vorrichtung 1 weist dabei eine Einrichtung zur Erfassung der Herztätigkeit auf, die in einem im Ausführungsbeispiel externen Herzschrittmacher 6 angeordnet ist, der über Herzdrähte 7 an das Herz 5 angeschlossen ist. Der Herzschrittmacher 6 weist seinerseits ein Zusatzmodul 8 auf, das einen Impuls für die Steuerung des Ballons 3 erzeugt, der der vom Herzschrittmacher 6 erfaßten oder der vom Schrittmacher 6 durch Stimulation bestimmten Herzphase (insbesondere Fig.5 und 6) zeitlich zugeordnet ist.

Die schematisierte Darstellung der Fig.1 verdeutlicht dabei, daß der Herzschrittmacher 6 extern angeordnet und über Elektroden oder Herzdrähte 7 an der Oberfläche des Herzens 5 fixiert und andererseits mit der Steuerung 4 für den Ballon 3 verbunden ist. Dabei erkennt man in Fig.1 ferner eine Leitung 9, die den Ballon 3 mit der eigentlichen Ballonpumpe 10 verbindet, welche zum Auf- und Abblasen des Ballons 3 vorgesehen ist, deren Steuerung 4 durch den von dem Zusatzmodul 8 erzeugten Impuls beaufschlagbar ist, also zu ihrer jeweiligen Betätigung angeregt wird. Der von dem Herzschrittmacher 6 beaufschlagte Zusatzmodul 8 ist also in die Steuerung der gesamten Vorrichtung 1 integriert und könnte über eine Leitung mit dem Herzschrittmacher 6 verbunden sein. Fig.1 deutet an, daß dies gewissermaßen innerhalb des Herzschrittmachers 6 der Fall sein kann.

Dadurch ist es möglich, daß ein Impuls zur Betätigung des Ballons 3 durch vom Herzschrittmacher 6 erfaßte oder stimulierte Herztätigkeit ausgelöst wird.

Die Steuereinheit 4 enthält im Ausführungsbeispiel eine Triggereinheit 11, welche die vom Herzschrittmacher 6 erfaßten und durch den Zusatzmodul 8 gebildeten Steuerimpulse aufnimmt und verarbeitet, wobei die Steuereinheit 4 aufgrund dieser Impulse eine Antriebseinheit 12, die elektrisch oder pneumatisch betätigt sein kann, ansteuert, welche die eigentliche Pumpe 10 antreibt.

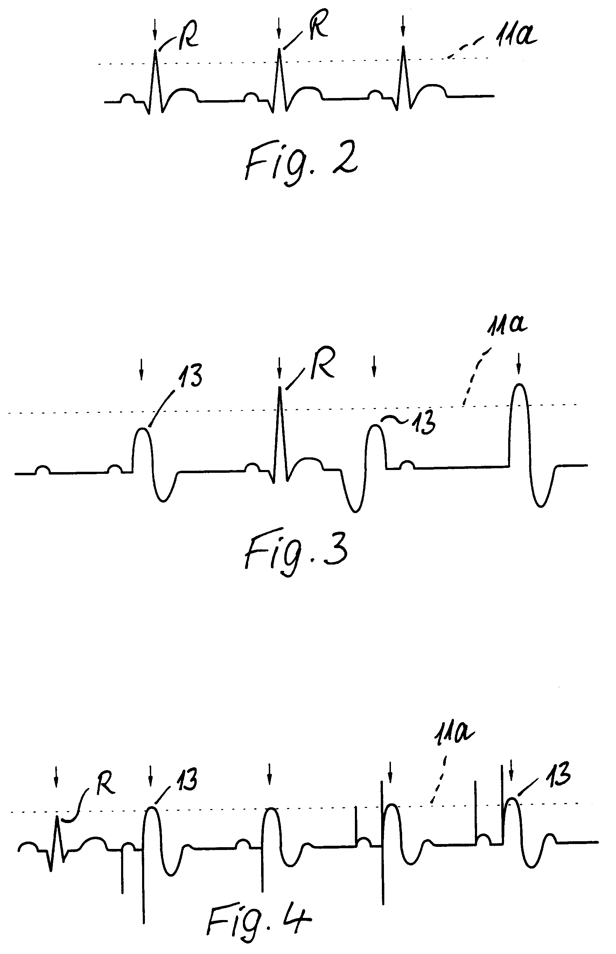

Bei einer EKG-Kurve gemäß Fig.3 mit spontan gestörtem Rhythmus (Extrasystolen) sowie einer EKG-Kurve gemäß Fig. 4 mit Schrittmachertätigkeit ist vom Oberflächen-EKG her keine vernünftige Triggerung der IABP möglich, weil verschiedenste Ereignisse ohne exakten Bezug zur Herztätigkeit zu unterschiedlichen Zeiten die Triggerlinie 11a überschreiten.

Die Lösung für das beschriebene Problem ist in den Figuren 5 und 6 dargestellt. Ein an sich rhythmusgestörtes Herz 5 wird dabei durch den externen Herzschrittmacher 6 "störungsfrei" gemacht und in einem Sonderkanal emittiert der Schrittmacher 6 ein EKG-artiges Signal, das R-wellenartig immer gleich aussieht und als zweite Kurve K unterhalb des eigentlichen EKG in Fig.5 und auch in Fig.6 dargestellt ist. Dieses EKG- und R-wellenartige Signal gelangt zu der Triggereinheit 11 und dadurch an den Pumpeneingang, wo keinerlei Schwierigkeit mit der Detektion oder Erkennung dieser Signale auftreten. Detektiert der Schrittmacher dabei ein "R" am Herzen, emittiert er das Pseudo-R sofort. Die natürlichen R-Wellen sind in Fig.5 und 6 mit einem "R" gekennzeichnet.

Stimuliert der Herzschrittmacher 6 ein "R" so emittiert er ein Pseudo-R PR gemäß der Kurve K versetzt um die Zeit t, die etwa 30 bis 50 Millisekunden beträgt. Die Pumpe 10 und der Ballon 3 werden also sowohl wahrgenommenen als auch stimulierten Kammerkomplexen (R) in der erforderlichen Zeit angekoppelt. Die Pseudo-R-Wellen für die IABP in den Kurven K überschreiten dabei jeweils den Trigger-Level 11a, so daß keine Störungen oder "Aussetzer" auftreten können.

Die Impulse PR gemäß den Kurven K, die von dem Zusatzmodul 8 am oder im Herzschrittmacher 6 erzeugt werden, können also wie das normale Oberflächen-EKG der Triggereinheit 11 über eine Verbindung 14 zugeführt werden und bewirken die erforderliche zeitgerechte Betätigung der Pumpe 10 bzw. des Ballons 3 in Abhängigkeit der jeweiligen Herztätigkeit.

Eine zusätzliche oder andere Ausgestaltung könnte darin bestehen, daß ein die Ballonpumpe 10 steuernder Prozessor dadurch gleichzeitig selbst als Herzschrittmacher ausgebildet ist, daß die Herzschrittmacherlogik in diesem Prozessor integriert ist. Um so einfacher ist die Handhabung durch das Personal.

Der externe Herzschrittmacher 6, welcher Mittel zur Stimulation des Herzens und Mittel zur Wahrnehmung spontaner Herztätigkeit hat, enthält eine zusätzliche Einrichtung in Form eines Zusatzmoduls 8 zur Bildung eines Impulses PR mit fester zeitlicher Koppelung an die Wahrnehmung spontaner ventrikulärer Herztätigkeit und fester zeitlicher, jedoch um einen bestimmten zeitlichen Betrag t verschobener Koppelung an die Abgabe eines ventrikulären Stimulationspulses, wobei dieser von dem Zusatzmodul 8 ausgehende Impuls zur Steuerung der Logik einer Ballonpumpe 10, 12, 9, 3 herangezogen wird. Der zusätzlich gebildete Impuls hat dabei etwa Form und Amplitude einer natürlichen, an der Körperoberfläche auftretenden R-Welle des EKG des Patienten, damit er vom EKG-Eingang der Pumpensteuerung 4 "verstanden" wird, wie das klassischerweise für das normalerweise abgeleitete Oberflächen-EKG vorgesehen ist. Er besitzt eine niedrige Quellimpedanz.

## Patentansprüche

1. Vorrichtung (1) zur Unterstützung der Herztätigkeit mit Hilfe eines in die Hauptschlagader (2) eingelegten aufblasbaren und abblasbaren Ballons (3) und einer Steuerung für eine Pumpe (10) zum Aufblasen und Abblasen dieses Ballons (3) in Übereinstimmung mit der Herztätigkeit derart, daß die durch das Aufblasen im Blutkreislauf erzeugte Druckwelle das Herz (5) kurz nach dem Schluß der Aortenklappe erreicht und daß die durch das Abblasen des Ballons (3) bewirkte negative Druckwelle die Aortenklappe zu dem Zeitpunkt erreicht, zu dem sie sich öffnet, wobei die Vorrichtung (1) eine Einrichtung zur Erfassung der Herztätigkeit sowie eine Steuerung zur Synchronisation der Vorrichtung mit dem aktuellen Herzrhythmus aufweist, wobei die Einrichtung zur Erfassung der Herztätigkeit in einem externen Herzschrittmacher (6) angeordnet ist, der über Elektroden oder Herzdrähte (7) an dem Herzen (5) angeschlossen oder fixiert ist, **dadurch gekennzeichnet**, daß die Einrichtung ein Zusatzmodul (8) aufweist, das einen zusätzlichen Impuls (PR) für die Steuerung (4) des Ballons (3) erzeugt, der etwa Form und/oder Amplitude einer natürlichen, an der Körperoberfläche auftretenden R-Welle hat und über den EKG-Eingang einer Triggereinheit (11) der Steuerung (4) der Pumpe (10) zuführbar ist, wobei dieser zusätzlich gebildete Impuls (PR) der von dem Herzschrittmacher (6) erfaßten oder der von dem Herzschrittmacher (6) durch Stimulation bestimmten Herzphase zeitlich zugeordnet ist.

2. Vorrichtung nach Anspruch 1, dadurch gekennzeichnet, daß der zusätzlich gebildete Impuls (PR) eine niedrige Quellimpedanz aufweist.

3. Vorrichtung nach Anspruch 1 oder 2, dadurch gekennzeichnet, daß der von dem Herzschrittmacher (6) beaufschlagte Zusatzmodul (8) in die Steuerung (4) der Vorrichtung (1) integriert und über eine Leitung (14) mit dem Herzschrittmacher (6) verbunden ist.

4. Vorrichtung nach einem der Ansprüche 1 bis 3, dadurch gekennzeichnet, daß der Impuls bei Wahrnehmung einer spontanen Herzkammertätigkeit sofort und bei Stimulation der Kammer zeitversetzt, vorzugsweise um etwa 50 Millisekunden, versetzt erzeugbar ist.

## Claims

1. An apparatus (1) to support heart activity with the help of an inflatable and deflatable balloon (3), which is disposed in the main artery (2), and a control system for a pump (10) for inflation and deflation of this balloon (3) in accordance with the cardiac activity in such a way that the pressure wave generated by the inflation action in the blood circulation reaches the heart (5) shortly after closure of the aortic valve, and that the negative pressure wave generated by deflation of the balloon (3) reaches the aortic valve at the point in time when it opens, wherein the apparatus (1) has equipment for recording cardiac activity and a control system for synchronisation of the apparatus with the current heart rhythm, wherein the equipment for recording the cardiac activity is arranged in an external cardiac pacemaker (6) which is connected or fixed to the heart (5) via electrodes or cardiac leads (7), characterised in that the equipment has an additional module (8) which generates an additional pulse (PR) for the control system (4) of the balloon (3), which has approximately the form and/or the amplitude of a natural R-wave which occurs on the body surface, and can be supplied to the control system (4) of the pump (10) via the ECG input of a trigger unit (11), wherein this additionally-formed pulse (PR) is temporally associated with the pulse recorded by the cardiac pacemaker (6) or that which is determined by the cardiac pacemaker (6) by stimulation.

2. An apparatus according to claim 1, characterised in that the additionally-formed pulse (PR) has a low source impedance.

3. An apparatus according to claim 1 or 2, characterised in that the additional module (8) which is acted upon by the cardiac pacemaker (6) is integrated into the control system (4) of the apparatus (1) and is connected to the cardiac pacemaker (6) via a cable (14).

4. An apparatus according to one of claims 1 to 3, characterised in that the pulse on detection of spontaneous heart ventricle activity can be generated immediately and, on stimulation of the ventricle, temporally offset, preferably by approximately 50 milliseconds.

## Revendications

1. Appareil (1) pour soutenir l'activité cardiaque à l'aide d'un ballonnet (3) gonflable et dégonfable placé dans l'artère principale (2) et d'une commande pour une pompe (10) servant à gonfler et à dégonfler ce ballonnet (3) en concordance avec l'activité cardique, de telle sorte que l'onde de pression créée dans la circulation sanguine par le gonflage atteint le coeur (5) peu après la fermeture de la valvule aortique et que l'onde de pression négative produite par le dégonflage du ballonnet (3) atteint la valvule aortique au moment où elle s'ouvre, appareil (1) qui comprend un dispositif pour détecter l'activité cardiaque ainsi qu'une commande pour synchroniser l'appareil avec le rythme cardiaque actuel, le dispositif pour détecter l'activité cardiaque étant agencé dans un stimulateur cardiaque (6) externe raccordé ou fixé au coeur (5) par des électrodes ou des connexions cardiaques (7), **caractérisé en ce que** l'appareil comporte un module additionnel (8) qui génère une impulsion supplémentaire (PR) pour la commande (4) du ballonnet (3), impulsion qui possède à peu près la forme et/ou l'amplitude d'une onde R naturelle apparaissant à la surface corporelle et peut être appliquée à travers l'entrée d'ECG d'une unité de déclenchement (11) à la commande (4) de la pompe (10), cette impulsion (PR), formée en plus, étant coordonnée dans le temps à la phase de pulsation cardiaque détectée par le stimulateur cardiaque (6) ou déterminée par lui par stimulation.

2. Appareil selon la revendication 1, caractérisé en ce que l'impulsions (PR), formée en plus, présente une basse fréquence de source.

3. Appareil selon la revendication 1 ou 2, caractérisé en ce que le module additionnel (8), sur lequel agit le stimulateur cardiaque (6), est intégré dans la commande (4) de l'appareil (1) et relié par une ligne (14) à ce stimulateur (6).

4. Appareil selon une des revendications 1 à 3, caractérisé en ce que l'impulsion peut être générée immédiatement à la perception d'une activité ventriculaire spontanée, et avec un décalage dans le temps, de préférence d'environ 50 millisecondes, en cas de stimulation du ventricule.
